# FASCICULE DE BREVET EUROPEEN

(11) **EP 1 052 978 B1**
(45) Date de publication et mention de la délivrance du brevet: **09.01.2002**
(21) Numéro de dépôt: 99903727.8
(22) Date de dépôt: 09.02.1999
(51) Int. Cl.: A61K 9/50

(54) **SYSTEME D'ENCAPSULATION A COEUR ORGANIQUE ET A ECORCE MINERALE A BASE D'HYDROXYCARBONATE D'ALUMINIUM ET SON PROCEDE DE PREPARATION**
EINKAPSELUNGSYSTEME MIT ORGANISCHEM KERN UND MINERALER UMHÜLLUNG ENTHALTEND ALUMINIUMHYDROXYCARBONAT UND VERFAHREN ZUR HERSTELLUNG
ENCAPSULATION SYSTEM WITH ORGANIC CORE AND MINERAL CORTEX BASED ON ALUMINIUM HYDROXYCARBONATE AND PREPARATION METHOD

(30) Priorité: 13.02.1998 FR 9801781
(43) Date de publication de la demande: 22.11.2000
(73) Titulaire: RHODIA CHIMIE, 92408 Courbevoie Cédex (FR)
(72) Inventeur: DUPUIS, Dominique, 94170 Deuil-la-Barre (FR)
(74) Mandataire: Le Coupanec, Pascale
(86) Numéro de dépôt international: FR9900280
(87) Numéro de publication internationale: WO9940902

(56) Documents cités:
- EP-A- 0 465 235
- FR-A- 2 401 619

## Description

La présente invention a pour objet un nouveau système d'encapsulation possédant une surface externe de nature essentiellement minérale et adaptée à la protection et/ou au relargage contrôlé de composé(s) organique(s).

Parmi les systèmes d'encapsulation déjà disponibles pour l'inclusion de substances organiques, on peut en distinguer deux types principaux :
- le premier système s'apparente à un système dit réservoir ; l'agent à encapsuler y est retenu à l'aide d'une membrane classiquement de nature polymérique. Ces capsules organiques sont obtenues par réaction interfaciale. La taille des capsules est généralement largement supérieure au micron ;
- le second système est dit système matriciel ; l'agent à encapsuler y est dispersé dans un réseau organique (polymère) ou dans une substance de nature minérale (en particulier silice). A titre illustratif de ce second système, on peut notamment citer les formulations phytosanitaires pour le relargage contrôlé d'insecticides avec des microcapsules de silice (Zairyo gijutsu, Vol. 5, n° 5 (1987) 231-236). Ces microcapsules de quelques microns sont préparées par précipitation d'un silicate alcalin en présence d'une émulsion eau dans huile, les particules étant ensuite calcinées. Les surfaces spécifiques des microcapsules sont très élevées (comprises entre 200 à 400 m²/g). Le principe actif est introduit par adsorption sur la poudre avec un taux d'adsorption relativement faible, de l'ordre de 10 %.

La présente invention a précisément pour objet de proposer un nouveau système d'encapsulation particulièrement avantageux au regard des systèmes évoqués ci-dessus.

L'encapsulation obtenue selon la présente invention a pour premier intérêt de protéger les principes actifs encapsulés de l'agressivité de l'environnement chimique par des capsules bio- ou écocompatibles. Enfin, elle a surtout pour avantage de permettre leur relargage soit par diffusion soit par dissolution de la coque, sous l'effet d'un changement de pH.

Plus précisément, le système d'encapsulation revendiqué met à profit la capacité de l'hydroxycarbonate d'aluminium à ne se solubiliser qu'à partir d'un pH inférieur à 5. II garantit ainsi dans tout milieu hôte de pH supérieur à 5, une encapsulation efficace aux composés organosolubles qu'il encapsule. L'hydroxycarbonate aluminium a en outre l'avantage de posséder une charge cationique qui peut être modulée en fonction du pH de précipitation. Il s'avère donc possible de moduler les interactions de charges entre le coeur constituant le système d'encapsulation et son écorce. Ceci présente un intérêt en terme de limitation de l'agglomération des particules du système d'encapsulation et de qualité de dépôt.

L'encapsulation de ces principes actifs est réalisée selon l'invention par précipitation in situ de l'hydroxycarbonate d'aluminium, constituant en tout ou partie l'écorce minérale de la capsule, en présence du ou desdits principe(s) actif(s) à encapsuler.

Le problème, plus précisément posé et résolu selon la présente invention, portait sur la formation d'une capsule à écorce composée d'hydroxycarbonate d'aluminium et donc de nature minérale autour d'un coeur essentiellement organique. Il importait de trouver un système tensioactif efficace pour, le cas échéant, stabiliser la formulation en soi du principe actif présent au coeur de la capsule, et surtout, permettre l'adhésion entre le coeur de nature organique et l'écorce à base d'hydroxycarbonate d'aluminium à caractère inorganique.

L'utilisation d'un tensioactif non ionique composé d'au moins un segment à caractère hydrophile et d'au moins un segment à caractère hydrophobe a permis de répondre de manière satisfaisante à cette double exigence.

En conséquence, la présente invention a pour premier objet un système d'encapsulation comprenant un coeur organique et une écorce minérale caractérisé en ce que :
- le coeur est constitué en tout ou partie d'au moins un principe actif organique,
- l'écorce minérale est constituée en tout ou partie d'hydroxycarbonate d'aluminium et
- en ce que la cohésion dudit système d'encapsulation est réalisée à l'aide d'au moins un tensioactif non ionique et comprenant au moins un segment hydrophobe et au moins un segment hydrophile.

Au sens de la présente invention, on entend couvrir sous la définition tensioactif à segment(s) hydrophobe(s) et segment(s) hydrophile(s), tout tensioactif possédant soit des motifs ou des chaînes à caractère hydrophile ou hydrophobe.

A titre illustratif mais non limitatif de ces tensioactifs, on peut notamment citer les composés suivants :
- les alcools gras polyalkoxylés, comme les alcools gras polyéthoxylés,
- les alkylphénolpolyoxyalkylénés comme les di- ou tristyrylphénols,
- les alcools ou alkyléthers polyvinyliques et
- les copolymères blocs comme le polystyrène/poly(oxyde d'éthylène).

En ce qui concerne le degré de polymérisation du segment poly(oxyde alkylène) présent chez certains composés identifiés ci-dessus, il est généralement compris entre 2 et 50 moles et plus préférentiellement varie entre 3 et 25 moles.

De manière préférée, ce tensioactif possède en outre une valeur HLB (Hydrophile-Lipophile Balance) propice pour stabiliser le cas échéant l'émulsion ou la dispersion du ou des principe(s) actif(s) organique(s) encapsulé(s). Généralement, cette valeur HLB est supérieure à 6 et de préférence supérieure à 10.

Plus préférentiellement, le tensioactif non ionique considéré selon la présente invention est choisi parmi les alcools polyalkoxylés, les tristyryl phénols et les copolymères blocs.

A titre représentatif des copolymères blocs convenant à la présente invention, on citera tout particulièrement le copolymère polystyrène/poly(oxyde d'éthylène) commercialisé sous le nom UPSE 1030® par la société Goldschmidt.

Parmi les alcools gras polyalkoxylés, l'alcool polyéthoxylé commercialisé sous le nom Emulgapur LM755® conduit également à des résultats satisfaisants selon l'invention.

Généralement, ce tensioactif non ionique est présent dans le système d'encapsulation revendiqué en quantité suffisante pour assurer la cohésion dudit système. De préférence, il est présent à raison d'environ 1 à 3 % exprimé en poids par rapport aux composants organiques.

En ce qui concerne le principe actif encapsulé, il peut être incorporé au sein du système d'encapsulation tel quel c'est-à-dire sous sa forme native, c'est-à-dire solide ou liquide.

On peut également envisager de l'incorporer sous la forme d'une émulsion aqueuse. Dans ce cas particulier, il est présent au niveau de la phase discontinue. Cette émulsion aqueuse peut être notamment obtenue par incorporation du principe actif pur ou du principe actif préalablement dissous dans une huile appropriée de type huile de silicone par exemple.

Il est également possible d'incorporer le principe actif sous la forme d'une dispersion aqueuse.

Généralement, le principe actif en tant que tel ou préalablement dissous dans une huile, peut être dispersé ou émulsionné jusqu'à 70 % en poids de la phase aqueuse.

Dans ces deux cas, le principe actif organique est de préférence stabilisé au sein de la phase aqueuse préalablement à l'encapsulation.

Comme évoqué précédemment, le principe actif peut être stabilisé au sein de la dispersion ou de l'émulsion aqueuse par le tensioactif non ionique selon l'invention. Toutefois, on peut également envisager d'incorporer au sein de la dispersion ou émulsion, un ou plusieurs autres tensioactifs.

Ces agents tensioactifs annexes, notamment utiles pour disperser, émulsionner, solubiliser et/ou stabiliser les divers composés à encapsuler peuvent être anioniques, non-ioniques, cationiques, zwitterioniques ou amphotères.

A titre illustratif de ces composés, on peut plus particulièrement citer :
- les agents tensioactifs anioniques tels que les alkylesters sulfonates, les alkylsulfates, les alkylamides sulfates et les sels d'acides gras saturés ou insaturés ;
- les agents tensioactifs non-ioniques tels que les alkylphénol polyoxyalkylénés, les glucosamides, les glucamides, les glycérolamides dérivés de N-alkylamines, les alcools aliphatiques en C₈-C₂₂ polyoxyalkylénés, les produits résultant de la condensation de l'oxyde d'éthylène avec un composé hydrophobe ou résultant de la condensation de l'oxyde de propylène avec le propylène glycol, les oxydes d'amines, les alkylpolyglycosides et leurs dérivés polyoxyalkylénés, les amides gras en C₈-C₂₀ et les acides gras, les amides, les amines, les amidoamines éthoxylés ;
- les agents tensioactifs amphotères et zwitterioniques tels que ceux de type bétaïne comme les bétaïnes, les sulfo-bétaïnes, les amidoalkylbétaïnes, les alkylsultaïnes, les produits de condensation d'acides gras et d'hydroxylats de protéines, les cocoamphoacétates et cocoamphodiacétates, les alkylampho-propionates ou -dipropionates, les dérivés amphotères des alkylpolyamines.

En ce qui concerne, la nature des composés organiques susceptibles d'être encapsulés conformément à la présente invention, il peut s'agir de composés à intérêt thérapeutique de type vitamines, de composés utiles dans le domaine phytosanitaire de types insecticides, ou encore d'agents destinés à un emploi en cosmétique comme les parfums, les agents anti-UV par exemple, etc.

En ce qui conceme plus particulièrement l'hydroxycarbonate d'aluminium il constitue, en tout ou partie, l'écorce du système d'encapsulation revendiqué.

On peut en effet envisager de l'associer à au moins un oxyde métallique et/ou précurseur d'un oxyde métallique au niveau de l'écorce minérale.

Selon cette variante, le coeur organique est enrobé d'au moins deux couches minérales distinctes et consécutives, l'une à base d'hydroxycarbonate d'aluminium et l'autre à base d'au moins un oxyde métallique et/ou précurseur d'un oxyde métallique. Dans ce cas particulier, le(s) dépôt(s) d'oxyde(s) métallique(s) sont de préférence effectué(s) consécutivement au dépôt d'hydroxycarbonate d'aluminium.

L'association d'au moins un oxyde métallique à l'hydroxycarbonate d'aluminium a pour avantage d'optimiser la diffusion des substances actives à travers l'écorce minérale. En fait, cet avantage repose sur une optimisation de la porosité et/ou de la sensibilité de l'écorce au pH.

En ce qui concerne l'oxyde métallique susceptible d'être incorporé au niveau de l'écorce minérale du système d'encapsulation, il peut être choisi parmi les oxydes ou précurseurs d'oxydes de silicium, de titane, de zirconium, de zinc, de magnésium, d'yttrium, de cérium et leurs mélanges.

A titre d'oxyde métallique convenant tout particulièrement à la présente invention, on peut citer les oxydes minéraux qui sont de préférence choisis parmi les oxydes de silicium, de zirconium, de cérium et leurs mélanges.

Plus préférentiellement il s'agit d'une silice, une alumine, un silicoaluminate ou un de leurs mélanges.

La taille des particules du système d'encapsulation revendiqué peut être de l'ordre de quelques dizaines de microns. De manière préférée, elle est comprise entre environ 0,1 et 10 µm.

Le système d'encapsulation proposé selon l'invention possède généralement un rapport massique charge minérale/charge organique inférieur ou égal à 1.

La présente invention a également pour objet un procédé de préparation d'un système d'encapsulation possédant un coeur organique constitué en tout ou partie d'au moins un principe actif organique et une écorce minérale constituée en tout ou partie d'hydroxycarbonate d'aluminium caractérisé en ce qu'il comprend :
- la précipitation in situ, en milieu aqueux, d'hydroxycarbonate d'aluminium, à un pH supérieur à 5, sous agitation en présence d'au moins un tensioactif non ionique, possédant au moins un segment hydrophobe et au moins un segment hydrophile et d'au moins un principe actif organique,
- la récupération de ladite dispersion aqueuse ainsi formée et le cas échéant,
- sa formulation sous une forme sèche.

En ce qui concerne le tensioactif non ionique, il répond à la définition proposée précédemment dans le cadre de la description détaillée du système d'encapsulation revendiqué.

La précipitation de l'hydroxycarbonate d'aluminium est effectuée par mise en contact d'une solution aqueuse d'aluminate de sodium carbonaté et de chlorure d'aluminium.

En ce qui concerne cette étape de précipitation in situ, elle est bien entendu effectuée à une valeur de pH appropriée pour conduire à une précipitation de l'hydroxycarbonate d'aluminium. En conséquence, ce pH doit être supérieur à 5. Il est de préférence compris entre 6 et 8. Le cas échéant, il peut s'avérer nécessaire de réajuster la valeur de pH au cours de cette étape de précipitation.

La précipitation in situ de l'hydroxycarbonate d'aluminium est réalisée sous agitation. Elle est de préférence réalisée à température ambiante. Toutefois, cette température peut varier entre 25 à 70°C.

En ce qui concerne le principe actif organique, il est présent lors de la précipitation in situ de l'hydroxycarbonate d'aluminium soit sous sa forme native soit sous la forme d'une émulsion ou d'une dispersion aqueuse.

L'émulsion et la dispersion aqueuses du principe actif sont telles que définies précédemment.

De préférence, la charge minérale et la charge organique sont utilisées avec un rapport massique inférieur ou égal à 1.

En ce qui concerne la nature des différents composants et notamment le tensioactif non ionique ainsi que leurs quantités respectives retenues dans le cadre du procédé revendiqué, on se reportera aux éléments décrits précédemment lors de la description du système d'encapsulation revendiquée selon l'invention.

On peut également envisager de faire subir à la dispersion aqueuse obtenue à l'issue de la seconde étape du procédé revendiqué une nouvelle étape de précipitation in situ d'un oxyde métallique et/ou précurseur d'un oxyde métallique annexe. Il peut notamment s'agir d'un oxyde et/ou précurseur tel qu'identifié précédemment. De préférence cet oxyde métallique est de la silice.

Selon cette variante du procédé revendiqué, on obtient un système d'encapsulation comprenant une écorce minérale composée de deux couches minérales distinctes et consécutives, la couche interne étant à base d'hydroxycarbonate d'aluminium et la couche externe constituée d'au moins un oxyde métallique et/ou précurseur d'oxyde métallique.

La formulation sous une forme sèche des dispersions aqueuses obtenues à l'issue du procédé revendiqué peut être réalisée par toute méthode conventionnelle sous réserve que celle-ci n'affecte pas la stabilité du ou des composés actifs encapsulés.

Comme discuté précédemment, le système d'encapsulation revendiqué et celui susceptible d'être obtenu selon le procédé revendiqué, sont particulièrement avantageux pour la protection et le relargage contrôlé des principes actifs organosolubles qu'ils contiennent.

Ce relargage peut être réalisé soit par diffusion par les pores du système d'encapsulation, par dissolution du système d'encapsulation sous l'effet d'une diminution du pH à une valeur inférieure à 5 dans le milieu externe contenant ledit système ou plus classiquement par fracture dudit système d'encapsulation.

Le second mode de relargage est particulièrement avantageux pour les domaines industriels de type pharmaceutique ou phytosanitaire. Il permet de s'assurer de la stabilité de la formulation pour toute valeur de pH supérieure à 5 et par là même de moduler la diffusion du principe actif soit au niveau d'un site déterminé et caractérisé par un pH inférieur à 5 comme la cavité gastrique pour ce qui est des applications thérapeutique par exemple, ou à un moment déterminé par ajustement du pH du milieu hôte à une valeur inférieure en 5, comme au moment de l'emploi de la formulation phytosanitaire correspondante.

La présente invention a également pour objet l'utilisation d'un système d'encapsulation tel que défini ou obtenu selon l'invention pour la protection et/ou le relargage contrôlé de principe(s) actif(s) organosoluble(s).

Les exemples figurant ci-après sont présentés à titre illustratif et non limitatif de la présente invention.

### EXEMPLE 1 :

### Enrobage d'une émulsion de polydiméthylsiloxane (PDMS) par une écorce d'hydroxycarbonate d'aluminium.

### Description de l'émulsion (polydiméthylsiloxane) :

Emulsion d'une huile silicone (MIRASIL DM 500 000® ) stabilisée par un alcool éthoxylée (Emulgapur LM 755® ), Dodécyle Sulfate de Sodium SDS et Rhéozan® .
ES : 44 %
Viscosité : 1180 mPa.S
Taille : 2,6 microns (déterminé à l'aide d'un SYMPATEC® )
pH : 5,6

### Matières premières :

- Solution de chlorure d'aluminium (SPCA)

| | |
|---|---|
| densité | 1,52 |
| Titre en Al₂O₃ | 21,5 à 24,5 % |
| Titre en Na₂O | 17 à 19 % |

- Solution d'aluminate de sodium (SPCA)

| | |
|---|---|
| densité | 1,5 |
| oxyde d'aluminium | 24 % |
| oxyde de sodium | 19 % |

- Carbonate de sodium (Prolabo)

| | |
|---|---|
| Rectapur® | 99 % Na₂CO₃ |

### Composition globale du milieu réactionnel :

| | |
|---|---|
| Emulsion : | 50 g |
| Eau : | 443,5 g |
| Solution d'aluminate carbonaté | 43,5 g |
| Solution de chlorure d'aluminium | 32 g |

La solution d'aluminate carbonaté a été au préalable préparée à partir de :

| | |
|---|---|
| Solution d'aluminate de sodium | 81,75 g |
| eau épurée | 144,8 g |
| Carbonate de sodium | 60,0 g |

### Mode opératoire

Dans un réacteur muni d'une agitation, on introduit à 25 °C, 443,5 g d'eau et 50 g d'émulsion. Le pH est ajusté à 6,5 par ajout de quelques gouttes d'aluminate carboné. Après stabilisation du pH à 6,5 on ajoute de façon simultanée la solution d'aluminate de sodium (débit : 2 ml/mn) et la solution de chlorure d'aluminium (débit : 1,2 ml/mn). Quelques minutes après le début d'introduction des réactifs la suspension flocule, la dispersion est remise en suspension par ultrasons. La dispersion est transférée dans un réacteur muni d'une agitation, l'introduction des réactifs est alors poursuivie jusqu'à introduction complète.

En fin de précipitation, la dispersion obtenue est stable. Les particules sont lavées par centrifugation et redispersées en milieu aqueux. L'absence de relargage est caractéristique d'encapsulation.

La dispersion obtenue présente à pH 6 un potentiel Zêta de + 20 mV, l'émulsion de départ présente par contre un potentiel Zêta de -15 mV.

L'inversion de charge avant et après encapsulation indique clairement l'obtention d'une capsule à base d'hydroxycarbonate d'aluminium.

### EXEMPLE 2

### Encapsulation de RETINOL 10 CM (vitamine A) par une écorce d'hydroxycarbonate d'aluminium.

### Matières premières :

Rétinol 10 CM® (Vitamine A : 10 % dans une huile)
Tensioactif (émulsion) : Copolymère bloc polystyrène/polyoxyde d'éthyfène (UPSE 1030® commercialisé par la société Goldschmidt)
- Solution de chlorure d'aluminium (SPCA)

| | |
|---|---|
| densité | 1,52 |
| Titre en Al₂O₃ | 21,5 à 24,5 % |
| Titre en Na₂O | 17 à 19 % |

- Aluminate de sodium (SPCA)

| | |
|---|---|
| densité | 1,5 |
| oxyde d'aluminium | 24 % |
| oxyde de sodium | 19 % |

- Carbonate de sodium (Prolabo)

| | |
|---|---|
| Rectapur® | 99 % Na₂CO₃ |

- Dodécyl sulfate de sodium (Aldrich) 98 % SDS

### Préparation de la solution d'aluminate carbonaté :

| | |
|---|---|
| Solution d'aluminate | 20,75 g |
| eau épurée | 37 g |
| Carbonate de sodium | 15 g |

Le mélange est agité pendant 30 minutes, il devient alors limpide et prêt à l'emploi.

### Préparation de l'émulsion :

| | |
|---|---|
| RETINOL 10 CM® | 20 g (10 % massique/eau) |
| USPE 1030® | 1,2 g |
| Eau épurée | 178,8 g |

Les 2 phases en présence sont émulsionnées à l'aide d'un Ultraturax® . La taille des gouttelettes est de 2 microns. Le pH de l'émulsion est compris entre 4 et 5.

### Composition globale du milieu réactionnel :

| | |
|---|---|
| Emulsion | 200 g |
| Eau | 200 g |
| SDS | 0,1 g |
| Solution d'aluminate carbonaté | 72,75 g |
| Solution de chlorure d'aluminium | 50 g |

### Mode opératoire:

Dans un réacteur double enveloppe, muni d'une agitation (à 300 t/min) on introduit à 25 °C, 200 g d'eau épurée contenant 0,1 g de SDS et 200 g d'émulsion. Le pH est ajusté à 6,4 par ajout de quelques gouttes d'aluminate carbonaté. Après stabilisation du pH à 6,4, on ajoute de façon simultanée les solutions d'aluminate carbonaté (débit : 2,3 ml/min) et d'AlCl₃ à débit variable permettant de réguler le pH à 6,4. L'introduction des réactifs est poursuivie jusqu'à addition complète (agitation normale).

En fin de précipitation, la dispersion obtenue est stable. Les particules sont lavées par centrifugation et redispersées en milieu aqueux. Au bout de 6 semaines aucune trace d'huile n'est constatée à la surface.

La taille des particules finales est voisine de 10 microns ; l'extrait sec de la dispersion est de 11,73 %.

### EXEMPLE 3

### Encapsulation de vitamine A par de l'hydroxycarbonate d'aluminium et de la silice.

La première étape qui consiste en l'encapsulation de la vitamine A par précipitation de l'hydroxycarbonate d'aluminium, est décrite en exemple 2. L'encapsulation par de la silice s'effectue sur le produit issu de l'exemple 2.

### Matières premières :

- Retinol encapsulé par un hydroxycarbonate d'aluminium (Al₂O₃ 50 % huile) préparé en exemple 2 et de composition suivante :

| | |
|---|---|
| exprimé en Al₂O₃ | 3,67 % |
| Retinol 10 CM® | 7,33 % (Vitamine A 0,73 %) |

- Silicate de sodium Prolabo Rectapur®

| | |
|---|---|
| Densité | 1,33 |
| Rm | 3,33 |
| SiO₂ | 26 % |

- NaOH 1 mol/l
- H₂SO₄ 30 g/l

### Préparation de la solution de silicate de sodium :

| | |
|---|---|
| Silicate | 23 g |
| Eau épurée | 92 g |

### Composition globale du milieu réactionnel :

| | |
|---|---|
| Dispersion exemple 2 | 162 g (Extrait sec : 11,73 %) |
| Eau épurée | 432 g |
| Silicate dilué | 115 g |
| NaOH à 1 mole/l | 52 g |
| H₂SO4 à 30 g/l | 60 g |

### Mode opératoire :

Dans un réacteur double enveloppe à 25 °C, agité à 300 t/min, on introduit 162 g de dispersion de l'exemple 2 et 432 g d'eau épurée. On ajuste le pH à 9 avec 18 g de soude 1M. A pH 9, on ajoute simultanément le silicate dilué à 3 ml/min, la soude 1 M et l'acide sulfurique à 30 g/l avec un débit fixé à 1 ml/min (pour l'acide). L'introduction des composés basiques est asservie de façon à maintenir le pH constant pendant l'introduction des réactifs.

Après ajout complet des réactifs, on laisse le mélange sous agitation à température ambiante pendant une heure.

On effectue ensuite une séparation par centrifugation pendant 30 minutes à 4 500 t/min et redispersion en milieu aqueux.

Le surnageant ne présente pas de traces d'huile. On n'assiste donc à aucun phénomène de relargage.

Les particules peuvent être récupérées sous forme de poudre après séchage à 40 °C.

### EXEMPLE 4 : Encapsulation de parfum par de l'hydroxycarbonate d'aluminium.

### Matières premières :

- Concentré de parfum
- Huile silicone Rhône-Poulenc 47V20®
- Tensioactif : Copolymère bloc polystyrènelpolyoxyde d'éthylène (UPSE 1030® commercialisé par la société Goldschmidt)
- Solution d'aluminate de sodium (SPCA) :

| | |
|---|---|
| densité | 1,5 |
| Oxyde d'aluminium | 24 % |
| Oxyde de sodium | 19 % |

- Carbonate de sodium (Prolabo)

| | |
|---|---|
| Rectapur® | 99 % |

- Dodécyle sulfate de sodium (Aldrich)98 %

### Préparation de l'aluminate carbonaté :

voir exemple 2

### Préparation de l'émulsion :

| | |
|---|---|
| Parfum | 2 g |
| Huile 47V20 | 18 g |
| Tensioactif | 1,2 g |
| Eau épurée | 178,8 g |

voir exemple 2 pour le mode opératoire.

### Composition globale du mélange :

| | |
|---|---|
| Emulsion | 200 g |
| Eau épurée | 200 g |
| SDS | 0,1 g |
| Aluminate carbonaté | 72,75 g |
| AlCl₃ | 50 g |

### Mode opératoire :

Dans un réacteur double enveloppe, on introduit à température ambiante sous agitation 300 t/min, 200 g d'émulsion et 200 g d'eau épurée contenant 0,1 g de SDS. On ajuste le pH à 6,4 avec quelques gouttes d'aluminate carbonaté. Après stabilisation du pH, on introduit l'aluminate carbonaté à 2,3 ml/min en même temps que AlCl₃ à pH constant (6,4). Après ajout total des réactifs, on laisse mûrir le mélange à température ambiante et sous agitation pendant une heure.

La suspension finale est stable, on la sépare des eaux mères par centrifugation à 4 500 t/min pendant 30 minutes. Le gâteau obtenu est redispersé dans de l'eau épurée.

## Revendications

1. Système d'encapsulation comprenant un coeur organique et une écorce minérale **caractérisé en ce que** :
- le coeur est constitué en tout ou partie d'au moins un principe actif organique,
- l'écorce minérale est constituée en tout ou partie d'hydroxycarbonate d'aluminium et
- **en ce que** la cohésion dudit système d'encapsulation est réalisée à l'aide d'au moins un tensioactif non ionique et comprenant au moins un segment hydrophobe et au moins un segment hydrophile.

2. Système selon la revendication 1, **caractérisé en ce que** le tensioactif non ionique est choisi parmi :
- les alcools gras poiyalkoxylés, comme les alcools gras polyéthoxylés,
- les alkylphénolpolyoxyalkylénés comme les di- ou tristyrylphénols,
- les alcools ou alkyléthers polyvinyliques et
- les copolymères blocs comme le polystyrène/poly(oxyde d'éthylène).

3. Système selon la revendication 2, **caractérisé en ce que** le degré de polymérisation du segment poly(oxyde alkylène) est compris entre 2 et 50 moles.

4. Système selon l'une quelconque des revendications 1 à 3 **caractérisé en ce que** le tensioactif non ionique est choisi parmi les alcools polyalkoxylés, les tristyrylphénols et les copolymères blocs.

5. Système selon l'une quelconque des revendications 1 à 4 **caractérisé en ce que** le tensioactif non ionique possède une HLB supérieure à 6.

6. Système selon l'une quelconques des revendications 1 à 5 **caractérisé en ce que** le tensioactif non ionique est présent dans le système d'encapsulation à raison d'environ 1 à 3 % exprimé en poids par rapport aux composants organiques.

7. Système selon l'une des revendications 1 à 6 **caractérisé en ce que** le principe actif est incorporé sous sa forme native au coeur dudit système.

8. Système selon l'une des revendications 1 à 6 **caractérisé en ce que** le principe actif organique est incorporé sous la forme d'une émulsion ou d'une dispersion aqueuse.

9. Système selon la revendication 8 **caractérisé en ce que** l'émulsion comprend ledit principe actif solubilisé dans une huile de silicone.

10. Système selon l'une des revendications 8 et 9 **caractérisé en ce que** le principe actif est dispersé ou émulsionné jusqu'à 70 % en poids de la phase aqueuse.

11. Système selon l'une des revendications 7 à 10 **caractérisé en ce que** le principe actif est stabilisé au sein de la phase aqueuse avec au moins un tensioactif annexe.

12. Système selon la revendication 11 **caractérisé en ce que** le tensioactif annexe est choisi parmi
- les agents tensioactifs anioniques tels que les alkylesters sulfonates, les alkylsulfates, les alkylamides sulfates et les sels d'acides gras saturés ou insaturés ;
- les agents tensioactifs non-ioniques tels que les alkylphénol polyoxyalkylénés, les glucosamides, les glucamides, les glycérolamides dérivés de N-alkylamines, les alcools aliphatiques en C₈-C₂₂ polyoxyalkylénés, les produits résultant de la condensation de l'oxyde d'éthylène avec un composé hydrophobe ou résultant de la condensation de l'oxyde de propylène avec le propylène glycol, les oxydes d'amines, les alkylpolyglycosides et leurs dérivés polyoxyalkylénés, les amides gras en C₈-C₂₀ et les acides gras, les amides, les amines, les amidoamines éthoxylés ;
- les agents tensioactifs amphotères et zwitterioniques tels que ceux de type bétaïne comme les bétaïnes, les sulfo-bétaïnes, les amidoalkylbétaïnes, les alkylsultaïnes, les produits de condensation d'acides gras et d'hydroxylats de protéines, les cocoamphoacétates et cocoamphodiacétates, les alkylampho-propionates ou -dipropionates, les dérivés amphotères des alkylpolyamines.

13. Système selon l'une des revendications 1 à 12 **caractérisé en ce que** le principe actif est un composé à intérêt thérapeutique, phytosanitaire ou cosmétique.

14. Système selon l'une des revendications 1 à 13 **caractérisé en ce que** l'hydroxycarbonate d'aluminium est associé dans l'écorce minérale à au moins un oxyde métallique et/ou précurseur d'un oxyde métallique.

15. Système selon la revendication 13 **caractérisé en ce que** l'écorce minérale est constituée d'au moins deux couches minérales distinctes et consécutives, l'une à base d'hydroxycarbonate d'aluminium et l'autre à base d'au moins un oxyde métallique et/ou précurseur d'un oxyde métallique.

16. Système selon l'une des revendications 13 à 15, **caractérisé en ce que** l'oxyde métallique est choisi parmi les oxydes ou précurseurs d'oxydes de silicium, de titane, de zirconium, de zinc, de magnésium, d'yttrium, de cérium et leurs mélanges.

17. Système selon l'une des revendications 1 à 16 **caractérisé en ce qu'**il possède une taille de particules comprise entre environ 0,1 et 10 µm.

18. Système selon l'une des revendications 1 à 17, **caractérisé en ce qu'**il possède un rapport massique charge minérale/charge organique inférieur ou égal à 1.

19. Procédé de préparation d'un système d'encapsulation comprenant un coeur organique constitué en tout ou partie d'au moins un principe actif organique et une écorce minérale constituée en tout ou partie d'hydroxycarbonate d'aluminium **caractérisé en ce qu'**il comprend:
- la précipitation in situ, en milieu aqueux d'hydroxycarbonate d'aluminium, à pH supérieur à 5, sous agitation et en présence d'au moins un tensioactif non ionique possédant au moins un segment hydrophobe et au moins un segment hydrophile et d'au moins un principe actif organique,
- la récupération de ladite dispersion ainsi formée et le cas échéant,
- sa formulation sous une forme sèche.

20. Procédé selon la revendication 19 **caractérisé en ce que** la précipitation de l'hydroxycarbonate d'aluminium est effectuée par mise en contact d'une solution aqueuse d'aluminate de sodium carbonaté et de chlorure d'aluminium.

21. Procédé selon la revendication 19 ou 20 **caractérisé en ce que** le pH est compris entre 6 et 8.

22. Procédé selon l'une des revendications 19 à 21, **caractérisé en ce que** le tensioactif non ionique est tel que défini en revendication 2 à 6.

23. Procédé selon l'une des revendications 19 à 22, **caractérisé en ce que** le principe actif est présent, lors de la précipitation in situ de l'hydroxycarbonate d'aluminium, sous sa forme native ou sous la forme d'une émulsion ou d'une dispersion aqueuse.

24. Procédé selon la revendication 23 **caractérisé en ce que** l'émulsion ou la dispersion du principe actif est telle que définie en revendications 9 à 13.

25. Procédé selon l'une des revendications 19 à 24 **caractérisé** le principe actif est tel que défini en revendication 13.

26. Procédé selon l'une des revendications 19 à 25 **caractérisé en ce que** la charge minérale et la charge organique sont utilisées avec un rapport massique inférieur ou égal à 1.

27. Procédé selon l'une des revendications 19 à 26 **caractérisé en ce que** la dispersion obtenue à l'issue de la seconde étape subit une nouvelle étape de précipitation in situ d'un oxyde métallique et/ou précurseur d'un oxyde métallique.

28. Procédé selon la revendications 27 **caractérisé en ce que** l'oxyde métallique précipité est de la silice.

29. Utilisation d'un système sur l'une des revendications 1 à 18 ou susceptible d'être obtenue par le procédé défini selon l'une des revendications 19 à 28 pour la protection et/ou le relargage contrôlé de principe(s) actif(s) organosoluble(s), les méthodes de traitement thérapeutique du corps humain ou animal étant exclues.

## Patentansprüche

1. Einkapselungssystem, das einen organischen Kern und eine anorganische Umhüllung umfasst, **dadurch gekennzeichnet, dass**
- der Kern ganz oder teilweise aus mindestens einem organischen Wirkstoff besteht und
- die anorganische Umhüllung ganz oder teilweise aus Aluminiumhydroxycarbonat besteht und dass
- die Kohäsion des Einkapselungssystems mittels mindestens eines Tenside herbeigeführt wird, das nichtionisch ist und mindestens ein hydrophobes und mindestens ein hydrophiles Segment umfasst.

2. System nach Anspruch 1, **dadurch gekennzeichnet, dass** das nichtionische Tensid aus
- polyalkoxylierten Fettalkoholen wie den polyethoxylierten Fettalkoholen,
- Polyoxyalkylenalkylphenolen wie Di- oder Tristyryrylphenolen,
- Polyvinylalkylethern oder Polyvinylalkoholen und
- Blockcopolymeren wie Polystyrol/Polyethylenoxid
ausgewählt ist.

3. System nach Anspruch 2, **dadurch gekennzeichnet, dass** der Polymerisationsgrad des Polyalkylenoxidsegments 2 bis 50 mol beträgt.

4. System nach einem der Ansprüche 1 bis 3, **dadurch gekennzeichnet, dass** das nichtionische Tensid aus polyalkoxylierten Alkoholen, Tristyrylphenolen und Blockcopolymeren ausgewählt ist.

5. System nach einem der Ansprüche 1 bis 4, **dadurch gekennzeichnet, dass** das nichtionische Tensid einen HLB-Wert von über 6 besitzt.

6. System nach einem der Ansprüche 1 bis 5, **dadurch gekennzeichnet, dass** das nichtionische Tensid im Einkapselungssystem mit etwa 1 bis 3 Gew.% der organischen Bestandteile vorhanden ist.

7. System nach einem der Ansprüche 1 bis 6, **dadurch gekennzeichnet, dass** der Wirkstoff in seiner nativen Form in den Kern des Systems eingebaut ist.

8. System nach einem der Ansprüche 1 bis 6, **dadurch gekennzeichnet, dass** der organische Wirkstoff in Form einer Emulsion oder einer wässrigen Dispersion eingebaut ist.

9. System nach Anspruch 8, **dadurch gekennzeichnet, dass** die Emulsion den Wirkstoff in einem Siliconöl solubilisiert enthält.

10. System nach Anspruch 8 oder 9, **dadurch gekennzeichnet, dass** der Wirkstoff bis zu 70 Gew.% der wässrigen Phase dispergiert oder emulgiert ist.

11. System nach einem der Ansprüche 7 bis 10, **dadurch gekennzeichnet, dass** der Wirkstoff in der wässrigen Phase mit mindestens einem anhängenden Tensid stabilisiert ist.

12. System nach Anspruch 11, **dadurch gekennzeichnet, dass** das anhängende Tensid aus
- anionischen Tensiden wie Alkylestersulfonaten, Alkylsulfaten, Alkylamidsulfaten und Salzen gesättigter oder ungesättigter Fettsäuren,
- nichtionischen Tensiden wie Polyoxyalkylenalkylphenolen, Glucosamiden, Glucamiden, Glycerinamiden, N-Alkylaminderivaten, C₈- bis C₂₂-polyoxyalkylenierten aliphatischen Alkoholen, Produkten, die aus der Kondensation von Ethylenoxid mit einer hydrophoben Verbindung oder von Propylenoxid mit Propylenglykol resultieren, Aminoxiden, Alkylpolyglycosiden und deren Polyoxyalkylenderivaten, C₈- bis C₂₀-Fettsäureamiden, Fettsäuren, Amiden, Aminen und ethyoxylierten Amidoaminen und
- amphoteren und zwitterionischen Tensiden wie denjenigen vom Betain-Typ wie Betainen, Sulfobetainen, Amidoalkylbetainen, Alkylsultainen, Kondensationsprodukten von Fettsäuren mit Proteinhydroxylaten, Cocoamphoacetaten, Cocoamphodiacetaten, Alkylamphopropionaten oder -dipropionaten und amphoteren Derivaten der Polyalkylamine
ausgewählt ist.

13. System nach einem der Ansprüche 1 bis 12, **dadurch gekennzeichnet, dass** der Wirkstoff eine Verbindung mit therapeutischer, kosmetischer oder Pflanzenschutzwirkung ist.

14. System nach einem der Ansprüche 1 bis 13, **dadurch gekennzeichnet, dass** das Aluminiumhydroxycarbonat in der anorganischen Umhüllung an mindestens ein Metalloxid und/oder einen Vorläufer eines Metalloxids angelagert ist.

15. System nach Anspruch 13, **dadurch gekennzeichnet, dass** die anorganische Umhüllung aus mindestens zwei voneinander verschiedenen, aufeinander folgenden anorganischen Schichten besteht, wovon eine auf der Basis von Aluminiumhydroxycarbonat und die andere auf der Basis von mindestens einem Metalloxid und/oder einem Vorläufer eines Metalloxids ist.

16. System nach einem der Ansprüche 13 bis 15, **dadurch gekennzeichnet, dass** das Metalloxid aus einem Oxid oder Vorläufer eines Oxids von Silicium, Titan, Zirconium, Zink, Magnesium, Yttrium und Cer und deren Gemischen ausgewählt ist.

17. System nach einem der Ansprüche 1 bis 16, **dadurch gekennzeichnet, dass** es eine Teilchengröße von etwa 0,1 bis 10 µm besitzt.

18. System nach einem der Ansprüche 1 bis 17, **dadurch gekennzeichnet, dass** es ein Massenverhältnis von anorganischer Füllung/organischer Füllung von kleiner als oder gleich 1 besitzt.

19. Verfahren zur Herstellung eines Einkapselungssystems, das einen organischen Kern, der ganz oder teilweise aus mindestens einem organischen Wirkstoff besteht, und eine anorganische Umhüllung, die ganz oder teilweise aus Aluminiumhydroxycarbonat besteht, umfasst, **dadurch gekennzeichnet, dass** es
- die Ausfällung von Aluminiumhydroxycarbonat in Gegenwart mindestens eines nichtionischen Tensids, das mindestens ein hydrophobes Segment und mindestens ein hydrophiles Segment besitzt, und mindestens eines organischen Wirkstoffs *in situ* im wässrigen Medium bei einem pH-Wert von über 5 unter Rühren,
- die Gewinnung der so gebildeten Dispersion und gegebenenfalls
- ihre Formulierung in einer trockenen Form
umfasst.

20. Verfahren nach Anspruch 19, **dadurch gekennzeichnet, dass** die Ausfällung des Aluminiumhydroxycarbonats durch In-Berührung-Bringen einer wässrigen Lösung von Natriumaluminatcarbonat und Aluminiumchlorid durchgeführt wird.

21. Verfahren nach Anspruch 19 oder 20, **dadurch gekennzeichnet, dass** der pH-Wert 6 bis 8 beträgt.

22. Verfahren nach einem der Ansprüche 19 bis 21, **dadurch gekennzeichnet, dass** das nichtionische Tensid wie in den Ansprüchen 2 bis 6 definiert ist.

23. Verfahren nach einem der Anaprüche 19 bis 22, **dadurch gekennzeichnet, dass** der Wirkstoff beim Ausfällen des Aluminiumhydroxycarbonats in *situ* in seiner nativen Form oder in Form einer Emulsion oder einer wässrigen Dispersion vorhanden ist.

24. Verfahren nach Anspruch 23, **dadurch gekennzeichnet, dass** die Emulsion oder Dispersion des Wirkstoffs wie in den Ansprüchen 9 bis 13 definiert ist.

25. Verfahren nach einem der Ansprüche 19 bis 24, **dadurch gekennzeichnet, dass** der Wirkstoff wie im Anspruch 13 definiert ist.

26. Verfahren nach einem der Ansprüche 19 bis 25, **dadurch gekennzeichnet, dass** die anorganische und die organische Füllung mit einem Massenverhältnis von kleiner als oder gleich 1 verwendet werden.

27. Verfahren nach einem der Ansprüche 19 bis 26, **dadurch gekennzeichnet, dass** die nach der zweiten Stufe erhaltene Dispersion einer erneuten Stufe der Ausfällung eines Metalloxids und/oder Vorläufers eines Metalloxids *in situ* unterworfen wird.

28. Verfahren nach Anspruch 27, **dadurch gekennzeichnet, dass** das ausgefällte Metalloxid Siliciumdioxid ist.

29. Verwendung eines Systems nach einem der Ansprüche 1 bis 18 oder das in der Lage ist, durch das gemäß einem der Ansprüche 19 bis 28 definierte Verfahren erhalten zu werden, für den Schutz und/oder die kontrollierte Abgabe von organisch solubilisierbarem /solubilisierbaren Wirkstoff/en, wobei die Verfahren zur therapeutischen Behandlung des menschlichen oder tierischen Körpers ausgeschlossen sind.

## Claims

1. Encapsulation system comprising an organic core end a mineral cortex **characterised in that**
- the core consists wholly or partly of at least one organic active principle;
- the mineral cortex consists wholly or partly of aluminium hydroxycarbonate and
- the cohesion of said encapsulation system is ensured by means of at least one non-ionic surfactant and comprising at least one hydrophobic segment and at least one hydrophilic segment.

2. System according to claim 1, **characterised in that** the non-ionic surfactant is selected from:
- polyalkoxylated fatty alcohols, such as polyethoxylated fatty alcohols,
- alkyl phenol polyoxyalkylenes, such as the di- or tri-styrylphenols,
- polyvinylic alcohols or alkylethers and
- block copolymers such as polystyrene/ poly (ethylene oxide).

3. System according to claim 2 **characterised in that** the degree of polymerisation of the poly (alkylene oxide) segment lies between 2 and 50 moles.

4. , System according to any of claims 1 to 3 **characterised in that** the non-ionic surfactant is selected from the polyalkoxylated alcohols, tristyrylphenols and block copolymers.

5. System according to any of claims 1 to 4 **characterised in that** the non-ionic surfactant has an HLB of more than 6.

6. System according to any of claims 1 to 5 **characterised in that** the non-ionic surfactant is present in the encapsulation system in a proportion of 1 to 3% expressed in terms of weight in relation to the organic components.

7. System according to one of claims 1 to 6 **characterised in that** the active principle is incorporated in its native form in the core of said system.

8. System according to one of claims 1 to 6 **characterised in that** the organic active principle is incorporated in the form of an emulsion or of an aqueous dispersion.

9. System according to claim 8 **characterised in that** the emulsion comprises said active principle solubilised in a silicone fluid.

10. System according to one of claims 8 or 9 **characterised in that** the active principle is dispersed or emulsified up to 70% by weight of the aqueous phase.

11. System according to one of claims 7 to 10 **characterised in that** the active principle is stabilised within the aqueous phase by at least one additional surfactant.

12. System according to claim 11 **characterised in that** the additional surfactant is selected from
- anionic surfactant agents such as alkylestersulphonates, alkyl sulphates, alkylamide sulphates and saturated or unsaturated fatty acid salts;
- the non-ionic surfactants such as polyoxyalkylenised alkylphenols, glucosamides, glucamides, glycerolamides derived from N-alkylamines, polyoxyalkylenised C₈-C₂₂ aliphatic alcohols in products created by the condensation of ethylene oxide with a hydrophobic segment or created by the condensation of propylene oxide with propylene glycol, amino-oxides, alkylpolyglycosides and their polyoxyalkylenised derivatives, C₈-C₁₀ fatty amides and fatty acids, amides, amines, and ethoxylated amidoamlnes;
- amphoteric and zwitterionic surfactant agents such as those of the betaine type, such as the betaines, sulpho-betaines, amidoalkylbetaines, alkylsulphaines, products of condensation of fatty acids and protein hydroxylates, cocoamphoacetates and cocamphodiacetates, alkylampho-propionates or -dipropionates, and the amphoteric derivatives of the alkylpolyamines.

13. System according to one of claims 1 to 12 **characterised in that** the active principle is a component of therapeutic, phytopathological or cosmetic importance.

14. System according to one of claims 1 to 13 **characterised in that** the aluminium hydroxycarbonate is associated in the mineral cortex with at least one metallic oxide and/or precursor of a metallic oxlde.

15. System according to claim 13 **characterised in that** the mineral cortex consists of at least two distinct and consecutive mineral layers, one based on aluminium hydroxycarbonate and the other based on at least one metallic oxide and/or precursor of a metallic oxide.

16. System according to one of claims 13 to 15 **characterised in that** the metallic oxide is selected from the oxides and/or precursors of oxides of silicon, titanium, zirconium, zinc, magnesium, yttrium, cerium and their blends.

17. System according to one of claims 1 to 16, **characterised in that** it has a particle size of between 0.1 and 10 m.

18. System according to one of claims 1 to 17, **characterised in that** it has a mineral filler / organic filler mass ratio of less than or equal to 1.

19. Method for preparation of an encapsulation system comprising an organic core consisting wholly or partly of at least one organic active principle and a mineral cortex consisting wholly or partly of aluminium hydroxycarbonate **characterised in that** it comprises:
- precipitation, in situ, in an aqueous medium of aluminium hydroxycarbonate with a pH of more than 5, under agitation and in the presence of at least one non-ionic surfactant having at least one hydrophobic segment and at least one hydrophilic segment and at least one organic active principle,
- the recovery of said dispersion thus formed and as applicable,
- its formulation in dry form.

20. Method according to claim 19 **characterised in that** the precipitation of the aluminium hydroxycarbonate is realised by the mixing of an aqueous solution of carbonated sodium aluminate with aluminium chloride.

21. Method according to claim 19 or 20, **characterised in that** the pH lies between 6 and 8.

22. Method according to one of claims 19 to 21, **characterised in that** the non-ionic surfactant is as defined in claims 2 to 6.

23. , Method according to one of claims 19 to 22, **characterised in that** the active principle is present, during the precipitation in situ of the aluminium hydroxycarbonate, in its native form or in the form of an emulsion or of an aqueous dispersion.

24. Method according to claim 23, **characterised in that** the emulsion or the dispersion of the active principle is as defined in claims 9 to 13.

25. Method according to one of claims 19 to 24, **characterised in that** the active principle is as defined in claim 13.

26. Method according to one of claims 19 to 25, **characterised in that** the mineral filler and the organic filler are used with a mass ratio less than or equal to 1.

27. Method according to one of claims 29 to 26, **characterised in that** the dispersion obtained at the end of the second stage undergoes a new stage of precipitation in situ of a metallic oxide and/or precursor of a metallic oxide.

28. Method according to claim 27, **characterised in that** the precipitated metallic oxide is silica.

29. Use of a system based on one of claims 1 to 18 or which it is possible to obtain by the method defined in accordance with one of claims 19 to 28 for the protection and/or controlled salting-out of organosoluble active principle(s), with the exclusion of therapeutic methods of treatment of the human or animal body.
